# FASCICULE DE BREVET EUROPEEN

(11) **EP 3 618 802 B1**
(45) Date de publication et mention de la délivrance du brevet: **22.01.2025**
(21) Numéro de dépôt: 18728212.4
(22) Date de dépôt: 03.05.2018
(51) Int. Cl.: A61P 17/00, A61K 8/9789, A61K 36/77, A61Q 19/00, A61Q 19/08, A61Q 19/06

(54) **UTILISATION D'UN EXTRAIT DE NEPHELIUM LAPPACEUM POUR AUGMENTER LA FERMETÉ DE LA PEAU ET/OU DES MUQUEUSES**
VERWENDUNG EINES NEPHELIUM-LAPPACEUM-EXTRAKTES ZUR ERHÖHUNG DER FESTIGKEIT DER HAUT UND/ODER DER SCHLEIMHÄUTE
USE OF A NEPHELIUM LAPPACEUM EXTRACT FOR INCREASING THE FIRMNESS OF THE SKIN AND/OR OF THE MUCOUS MEMBRANES

(30) Priorité: 05.05.2017 FR 1753987
(43) Date de publication de la demande: 11.03.2020
(73) Titulaire: BASF Beauty Care Solutions France SAS, 69007 Lyon (FR)
(72) Inventeur: BARDEY, Vincent, 54000 Nancy (FR); BONNET, Isabelle, 69008 Lyon (FR); ECHARD, Anabelle, 54300 Luneville (FR); PELLETIER, Nicolas, 69004 Lyon (FR); VOGELGESANG, Boris, 69002 Lyon (FR)
(74) Mandataire: Cabinet Becker et Associés
(86) Numéro de dépôt international: PCT/FR2018/051095
(87) Numéro de publication internationale: WO 2018/203000

(56) Documents cités:
- WO-A1-2010/014861
- WO-A1-2014/170347
- FR-A1- 2 941 373
- JP-A- 2011 006 333
- KR-A- 20090 056 521
- NATTAYA LOURITH ET AL: "In vitro and cellular activities of the selected fruits residues for skin aging treatment", ANAIS DA ACADEMIA BRASILEIRA DE CIÊNCIAS, vol. 89, no. 1 suppl, 1 May 2017 (2017-05-01), pages 577 - 589, XP055430051, DOI: 10.1590/0001-3765201720160849
- ARIFFIN NOR HAZWANI ET AL: "Potential dermatological application on Asian plants", BIOTECHNOLOGY AND BIOPROCESS ENGINEERING, KOREAN SOCIETY FOR BIOTECHNOLOGY AND BIOENGINEERING, SEOUL, KR, vol. 21, no. 3, 22 July 2016 (2016-07-22), pages 337 - 354, XP036010982, ISSN: 1226-8372, [retrieved on 20160722], DOI: 10.1007/S12257-015-0750-4
- DATABASE GNPD [online] MINTEL; 1 August 2011 (2011-08-01), TONYMOLY: "Hot Body Gel", XP002776210, Database accession no. 1606102
- DATABASE GNPD [online] MINTEL; 1 June 2010 (2010-06-01), TONYMOLY: "Eye Cream", XP002776211, Database accession no. 1349546
- DATABASE GNPD [online] MINTEL; 1 February 2017 (2017-02-01), GLAMGLOW: "Matte Lip Treatment", XP002776212, Database accession no. 4635695
- MAHENDRAN SEKAR ET AL: "FORMULATION AND EVALUATION OF NOVEL ANTIAGING CREAM CONTAINING RAMBUTAN FRUITS EXTRACT", INTERNATIONAL JOURNAL OF PHARMACEUTICAL SCIENCES AND RESEARCH IJPSR, 1 January 2017 (2017-01-01), pages 1056 - 1065, XP055430061, Retrieved from the Internet <URL:http://ijpsr.com/bft-article/formulation-and-evaluation-of-novel-antiaging-cream-containing-rambutan-fruits-extract/?view=fulltext> [retrieved on 20171129]
- PALANISAMY UMA ET AL: "Rind of the rambutan,Nephelium lappaceum, a potential source of natural antioxidants", FOOD CHEMISTRY, ELSEVIER LTD, NL, vol. 109, no. 1, 23 December 2007 (2007-12-23), pages 54 - 63, XP029181558, ISSN: 0308-8146, DOI: 10.1016/J.FOODCHEM.2007.12.018
- EUIS RENI YUSLI ET AL: "Effect of Rambutan-honey and its Flavonoid on TGF-[beta]1 Induce Fibroplasia Oral Wound Healing", RESEARCH JOURNAL OF MEDICINAL PLANT, vol. 10, no. 8, 15 October 2016 (2016-10-15), US, pages 435 - 442, XP055430065, ISSN: 1819-3455, DOI: 10.3923/rjmp.2016.435.442
- C ORWA ET AL: "Nephelium lappaceum (Rambutan)", AGROFORESTREE DATABASE:A TREE REFERENCE AND SELECTION GUIDE VERSION 4.0, 1 January 2009 (2009-01-01), pages 1 - 5, XP055429429, Retrieved from the Internet <URL:http://www.worldagroforestry.org/treedb/AFTPDFS/Nephelium_lappaceum.PDF> [retrieved on 20171128]
- ZANETTI M ET AL: "EMILIN-1 DEFICIENCY INDUCES ELASTOGENESIS AND VASCULAR CELL DEFECTS", MOLECULAR AND CELLULAR BIOLOGY, AMERICAN SOCIETY FOR MICROBIOLOGY, WASHINGTON, US, vol. 24, no. 2, 1 January 2004 (2004-01-01), pages 638 - 650, XP001184025, ISSN: 0270-7306, DOI: 10.1128/MCB.24.2.638-650.2004

## Description

La présente invention concerne le domaine de la cosmétique et de la dermatologie, en particulier la nouvelle utilisation cosmétique et/ou dermatologique d'un extrait de la plante *Nephelium lappaceum* pour augmenter la fermeté et/ou l'élasticité de la peau et/ou des muqueuses.

Le collagène est une protéine constitutive majeure de la matrice extra-cellulaire (MEC) présente en grande quantité dans les tissus vertébrés. Il fait partie d'une superfamille de 29 membres distincts, dont les collagènes fibrillaires. Le collagène de type I en est l'exemple le plus connu mais le collagène de type V est aussi un collagène fibrillaire. On retrouve le collagène de type V dans les mêmes tissus que le collagène de type I, et il est connu pour aider à l'assemblage des fibres hétérogènes composées de ces 2 types de collagène. Ils assurent donc le maintien des structures et confèrent aux tissus leur résistance mécanique. Ainsi au niveau du derme, il contribue à la fermeté de ces tissus, donc à la fermeté de la peau.

Au cours du vieillissement intrinsèque des tissus, particulièrement du derme et de l'épiderme, l'expression du collagène diminue, induisant un relâchement de ces tissus, donc une perte de tonicité et de fermeté de la peau. Différents facteurs extrinsèques sont également responsables de perte de fermeté de la peau et/ou des muqueuses notamment les agents agressifs de l'environnement tels que les rayonnements UV, la pollution, le tabac. D'autres facteurs intrinsèques peuvent également être responsables de désorganisation de fibres de collagène et d'une diminution de fermeté notamment les variations hormonales, et les variations de tensions au niveau de la peau et/ou des muqueuses (prise ou perte de poids rapides).

La fibrilline-1 quant à elle est aussi une protéine synthétisée par les fibroblastes, constitutive de la MEC au niveau du derme. Elle participe à la formation des fibres élastiques constituées d'élastine. Tout comme le collagène, l'expression de cette protéine diminue au cours du vieillissement de la peau et/ou d'autres facteurs intrinsèques, notamment hormonal et de facteurs extrinsèques notamment de l'environnement, induisant un relâchement des tissus élastiques.

Une autre protéine participe à la formation des fibres élastiques, LOX-L (Lysyl oxidase-like). Cette enzyme permet la réticulation de la tropoélastine, laquelle est ensuite déposée au niveau des microfibrilles. De même que le collagène et la fibrilline-1, son expression va diminuer avec le temps au niveau de la peau et des muqueuses.

On citera enfin les protéines CYR61 (Cysteine rich angiogenic inducer 61) régulant certaines synthèses de la MEC, la fibuline-5 constitutive des fibres élastiques ou encore l'Emiline-1, une glycoprotéine de la MEC associée aux fibres élastiques. De leur niveau d'expression vont dépendre la formation et l'agencement correct des fibres élastiques.

Ces protéines, collagène I, collagène V, fibrilline-1, CYR61, Emiline-1, fibuline-5 et LOX-L constituent par conséquent des cibles privilégiées dans le domaine de la cosmétique et de la dermatologie, domaines en demande constante d'ingrédients actifs alternatifs pour lutter contre la perte de fermeté et/ou d'élasticité.

De façon particulièrement surprenante, les inventeurs ont découvert qu'un extrait de la plante *Nephelium lappaceum* possède la propriété d'augmenter la fermeté de la peau et/ou des muqueuses, notamment en augmentant l'expression génique et/ou protéique de collagène de type I et/ou de type V, dans la peau et les muqueuses mais aussi d'augmenter l'élasticité de la peau et/ou des muqueuses, notamment en augmentant l'expression génique et/ou protéique de fibrilline-1, de LOX-L, de fibuline-5 et d'Emiline-1.

L'extrait selon l'invention est un extrait de la plante *N. lappaceum.* On trouve cet arbre, aussi appelée ramboutan, en Asie du sud-est, notamment en Malaisie et Indonésie. Il s'agit d'un arbre de 10 à 20 mètres de hauteur, produisant une grande quantité de fruits. Ce fruit, comestible, est connu pour ses propriétés organoleptiques, il contient des doses importantes de sucres, de vitamine C et de fer. Des décoctions de racines ou de feuilles séchées ont aussi été utilisées pour lutter contre la fièvre.

La provenance de la plante à partir de laquelle est préparé l'extrait selon l'invention est le Vietnam. L'extrait selon l'invention présente l'avantage d'être efficace sur plusieurs types de collagènes : le collagène de type I et de type V. L'extrait permet donc d'augmenter la fermeté de la peau et/ou des muqueuses en augmentant l'expression génique et/ou protéique de collagène de type I, tout en augmentant en complément l'expression d'autres composants de la MEC tels que le collagène de type V ou en diminuant l'expression de CYR61. Un autre avantage encore de l'extrait selon l'invention est qu'il permet d'augmenter l'élasticité de la peau et/ou des muqueuses en augmentant l'expression génique et/ou protéique de la fibrilline-1 mais aussi de LOX-L, de fibuline-5 et d'Emiline-1. Cet extrait permet donc de cibler plusieurs types de protéines de la MEC, en faisant un ingrédient cosmétique et/ou dermatologique complet.

L'extrait selon l'invention présente en outre l'avantage d'être un ingrédient facilement formulable, et pouvant être facilement fabriqué à l'échelle industrielle. Il s'agit d'un ingrédient actif topiquement acceptable pour la peau et les muqueuses, qui ne présente pas de risque d'allergie.

Un gel commercial pour le corps possédant un effet fermeté et comprenant 10 extraits de plantes, dont un extrait de *N. lappaceum,* a été décrit. Toutefois, l'ingrédient actif sur la fermeté est un extrait de poivre comprenant de la capsaicine et aucune activité sur la fermeté, en particulier choisie parmi la fermeté, une activité anti-cellulite et une activité amincissante, n'est associée à l'extrait de *N. lappaceum.*

La demande KR20060007083 décrit une composition cosmétique comprenant un extrait de *N. lappaceum,* ladite composition étant utilisée comme agent blanchissant de la peau.

Des extraits de la plante préparés par extraction dans différents solvants dont l'éthanol ont été décrits pour leur activité anti-radicalaire dans la demande WO2008066370. De même, la demande JP2002145730 divulgue plusieurs effets dont un effet hydratant et anti-oxydant des graines de la plante *N. lappaceum.*

Lourith et al. (2017, Anais da Academia Brasileira de Ciencias, 89, 577-589) décrit par ailleurs des extraits de péricarpe de litchi (*Litchi chinensis*) et de *N. lappaceum* comme possédant une activité anti-collagénase. Mais nulle part dans ce document n'est décrit l'utilisation d'un extrait de *N. lappaceum* pour augmenter la fermeté de la peau, ni pour augmenter l'expression de collagène de type I ou V en particulier. Il n'est pas non plus fait mention d'un extrait de feuilles de *N. lappaceum.* En outre, ce document fait état de l'utilisation préférentielle de l'extrait de litchee dans des produits cosmétiques anti-âge, plutôt que de l'extrait de *N. lappaceum.*

Ariffin Nor Hazwani et al. (Biotechnology and bioprocess engineering, Korean Society for Biotechnology and Bioengineering, Seoul, KR, vol. 21, no.3, 22 juillet 2016, pages 337-354*)* porte sur le rôle des plantes asiatiques en tant qu'agent protecteur de l'épiderme. Cet article divulgue en particulier l'effet cicatrisant et anti-infectieux d'un extrait aqueux de feuilles de ramboutan.

Enfin, la demande KR20090056521 décrit un extrait de *N. lappaceum* et de litchi (*Litchi chinensis*) dans une composition cosmétique pour la peau, possédant un effet anti-ride et permettant d'inhiber la dégradation du collagène par les métalloprotéinases (MMP), notamment les MMP-1. Toutefois, il n'est pas décrit dans cet art antérieur un effet d'augmentation de l'expression protéique et/ou génique de collagène de type I ni V par un extrait de *N. lappaceum,* en particulier par un extrait de feuilles. Il n'y est pas divulgué non plus un effet sur l'augmentation de la fermeté de la peau par cet extrait dans cette demande de brevet. Par ailleurs, elle décrit un effet d'inhibition de protéinases responsables d'une dégradation de collagène, mais pas d'une augmentation de l'expression de collagène de type I ni de type V en particulier. Ainsi à la connaissance de la demanderesse, aucun document ne décrit l'utilisation d'un extrait de la plante *N. lappaceum* pour augmenter la fermeté ni l'élasticité de la peau et/ou des muqueuses ni pour augmenter l'expression de collagène de type I et de type V, de fibuline-1, d'Emiline-1, de LOX-L et/ou de fibrilline, et/ou diminuer l'expression de CYR61, dans la peau et/ou les muqueuses.

L'invention est telle que définie dans les revendications.

Ainsi, l'invention concerne selon un premier objet l'utilisation cosmétique d'un extrait de *N. lappaceum* pour augmenter la fermeté de la peau et/ou des muqueuses.

L'invention concerne selon un second objet l'utilisation cosmétique d'un extrait de *N. lappaceum* pour augmenter l'élasticité de la peau et/ou des muqueuses. On distingue en effet au sens de l'invention une efficacité sur la fermeté, d'une efficacité sur l'élasticité de la peau et/ou des muqueuses. La fermeté est étroitement liée à la densité de la matrice extracellulaire, notamment mais non exclusivement à l'expression des collagènes. L'élasticité est liée à la formation et l'assemblage des fibres élastiques, auxquelles les protéines fibrilline-1, LOX-L, fibuline-5 et Emiline-1 participent.

On entend au sens de la présente invention par « utilisation cosmétique » une utilisation non thérapeutique, c'est-à-dire destinée à être appliquée sur tout ou partie d'une zone de peau ou de muqueuse saine, non pathologique. On entend par « zone de peau saine et/ou muqueuse saine », une zone de peau et/ou de muqueuse sur laquelle est appliquée l'extrait selon l'invention et qualifiée de « non pathologique » par un dermatologue, c'est à dire ne présentant pas d'infection, de cicatrice, de maladie ou d'affection cutanée telle que candidose, impétigo, psoriasis, eczéma, acné ou dermatite, ou de plaies ou de blessures et/ou autres dermatoses.

L'extrait selon l'invention peut être appliqué sur tout ou partie de peau du corps et/ou du visage où l'augmentation de fermeté et/ou d'élasticité est souhaitée, préférentiellement les jambes, les cuisses, les bras, le ventre, le décolleté, le cou, tout ou partie du visage, préférentiellement les joues, le front, le menton, les lèvres, le contour des lèvres, le contour des yeux, la zone dite « en T » du visage. Au sens de la présente invention, la peau inclut le cuir chevelu.

On entend au sens de la présente invention par « collagène » le collagène de type I, III, IV, V, VI, VII, XII, XIII, XIV, XVI, XVII, XVIII, XXIV et/ou XXIX présents dans la peau et/ou les muqueuses. Préférentiellement selon l'invention, il s'agit de collagène de type I et/ou V, encore préférentiellement le collagène de type I.

On entend par ailleurs par « fibrilline » la fibrilline-1, fibrilline-2 et/ou fibrilline-3, préférentiellement la fibrilline-1 présente au niveau de la peau et/ou des muqueuses.

L'extrait de *N. lappaceum* selon l'invention est un ingrédient topiquement acceptable. On entend par « topiquement acceptable », un ingrédient adapté à une application par voie topique, non toxique, non irritant pour la peau et/ou les muqueuses, n'induisant pas de réponse allergique, qui n'est pas instable sur le plan chimique. L'utilisation de l'extrait selon la présente invention peut être par voie orale ou topique. Avantageusement, elle est par voie topique. On entend par « voie topique », l'application locale directe et/ou la vaporisation de l'ingrédient sur la surface de la peau et/ou des muqueuses.

On entend par « muqueuse », la muqueuse oculaire, la muqueuse vaginale, la muqueuse urogénitale, la muqueuse anale, la muqueuse nasale et/ou la muqueuse buccale, labiale et/ou gingivale, préférentiellement, les muqueuses labiales et/ou buccales.

On entend au sens de la présente invention par « augmenter la fermeté de la peau et/ou des muqueuses » une augmentation de la fermeté au niveau de la peau et/ou des muqueuses d'au moins 1%, préférentiellement d'au moins 3%, encore avantageusement d'au moins 5% en présence d'un extrait de *N. lappaceum* selon l'invention. Dans un mode de réalisation avantageux de l'invention, il s'agit d'une augmentation mesurée in *vivo,* préférentiellement sur la peau du visage humain. Encore préférentiellement, cette augmentation de la fermeté de la peau du visage humain est mesurée après application d'une crème comprenant un extrait de feuilles de *N. lappaceum,* préférentiellement présent en quantité de 0,1%, en poids par rapport au poids total de la crème. Alternativement, l'extrait de feuilles sera présent en quantité de 2% en poids par rapport au poids total de la crème. Dans ce cas, il s'agira d'un extrait préparé dans l'eau en conditions subcritiques dans les conditions décrites dans l'exemple 1e).

Dans un mode avantageux de réalisation de l'invention, la crème est appliquée sur l'hémivisage d'une population de 30 femmes âgées de 55 à 65 ans, et la mesure de l'augmentation de fermeté est effectuée après 28 et 56 jours d'application quotidienne de ladite crème, en comparaison de l'application dans les mêmes conditions d'une crème placebo qui ne comprend pas l'extrait de feuilles de *N. lappaceum.*

La mesure in *vivo* de la fermeté pourra être effectuée selon les méthodes classiques connues de l'homme de l'art, notamment par mesure à l'aide d'un cutomètre, d'un Tonoderm^{™}, d'un DynaSKIN^{®} associé à un dermaTOP ou par l'intermédiaire d'un appareil nommé SkinFibroMeter (Delfin). Dans un mode particulièrement avantageux de réalisation de l'invention, la fermeté est évaluée par mesure de la répartition des volumes de la peau soumise à déformation sous pression du SkinFibroMeter, permettant d'évaluer les propriétés biomécaniques de la peau.

On entend en outre par « augmenter l'élasticité » de la peau et/ou des muqueuses une augmentation de l'élasticité mesurée *in vivo,* d'au moins 2%, avantageusement d'au moins 4%, encore avantageusement d'au moins 6% en présence de l'extrait selon l'invention par rapport à l'élasticité détectée en l'absence de l'extrait. Dans un mode de réalisation préférentiel de l'invention, cette mesure est effectuée sur la peau du visage humain. Encore préférentiellement, l'augmentation de l'élasticité de la peau du visage humain est mesurée en présence d'une crème comprenant un extrait de feuilles de *N. lappaceum,* avantageusement présent en quantité de 1% en poids par rapport au poids total de la crème. Alternativement, l'extrait de feuilles sera présent en quantité de 2% en poids par rapport au poids total de la crème. Dans ce cas, il s'agira d'un extrait préparé dans l'eau en conditions subcritiques dans les conditions décrites dans l'exemple 1e).

Dans un mode avantageux de réalisation de l'invention, la crème est appliquée sur l'hémivisage d'une population de 30 femmes âgées de 55 à 65 ans, et la mesure de l'élasticité est effectuée après 28 et 56 jours d'application quotidienne de ladite crème, en comparaison de l'application dans les mêmes conditions d'une crème placebo qui ne comprend pas l'extrait de feuilles *de N. lappaceum.* La mesure de l'élasticité pourra être effectuée à l'aide d'un ballistomètre, d'un cornéomètre ou d'un cutomètre. Dans un mode de réalisation avantageux, elle sera mesurée par cutométrie, technique permettant de mesurer la déformation mécanique de la peau soumise à succion.

La méthode dite de projection à franges permettra de mesurer *in vivo* les rides. Ainsi dans le cadre de la présente invention, on entend par « diminuer les rides » une diminution d'au moins 0,5%, préférentiellement d'au moins 1%, encore préférentiellement d'au moins 2% des fines rides en présence de l'extrait selon l'invention par rapport au niveau de fines rides mesuré en l'absence de l'extrait. Dans un mode de réalisation préférentiel de l'invention, cette mesure est effectuée sur la peau du visage humain. Encore préférentiellement, la diminution des fines rides de la peau du visage humain est mesurée en présence d'une crème comprenant un extrait de feuilles de *N. lappaceum,* avantageusement présent en quantité de 1% en poids par rapport au poids total de la crème. Alternativement, l'extrait de feuilles sera présent en quantité de 2% en poids par rapport au poids total de la crème. Dans ce cas, il s'agira d'un extrait préparé dans l'eau en conditions subcritiques dans les conditions décrites dans l'exemple 1e).

Dans un mode avantageux de réalisation de l'invention, la crème est appliquée sur l'hémivisage d'une population de 30 femmes âgées de 55 à 65 ans, et la mesure des fines rides est effectuée après 28 et 56 jours d'application quotidienne de ladite crème, en comparaison de l'application dans les mêmes conditions d'une crème placebo qui ne comprend pas l'extrait de feuilles de *N. lappaceum.*

Dans un mode avantageux de réalisation de l'invention, l'extrait de *N. lappaceum* n'est pas combiné à un quelconque extrait de la plante *L. chinensis.* De façon particulière, l'extrait selon l'invention n'est préférentiellement pas combiné à un extrait de fruit de *L. chinensis.*

De même, l'extrait selon l'invention n'est préférentiellement pas combiné à de la capsaicine (numéro CAS 404-86-4 ; masse molaire 305,418 g/mol) ou un quelconque extrait de plante en comprenant. De façon particulière, l'extrait selon l'invention n'est pas combiné à un extrait de fruit d'une quelconque espèce de plante du genre Capsicum comprenant de la capsaicine.

Un objet de la présente invention est donc l'utilisation cosmétique d'un extrait de *N. lappaceum* pour augmenter la fermeté et/ou l'élasticité de la peau et/ou des muqueuses, en augmentant l'expression génique et/ou protéique de collagène de type I, de type V, d'Emiline-1, de fibuline-5, de fibrilline, préférentiellement de fibrilline-1, et/ou de LOX-L, et/ou en diminuant l'expression de CYR61.

En particulier, un objet de la présente invention est donc l'utilisation cosmétique d'un extrait de *N. lappaceum* pour augmenter la fermeté de la peau et/ou des muqueuses, en augmentant l'expression génique et/ou protéique de collagène de type I et/ou de type V et/ou en diminuant l'expression de CYR61. Un autre objet particulier de la présente invention est l'utilisation cosmétique d'un extrait de *N. lappaceum* pour augmenter l'élasticité de la peau et/ou des muqueuses, en augmentant l'expression génique et/ou protéique d'Emiline-1, de fibuline-5, de fibrilline, préférentiellement de fibrilline-1, et/ou de LOX-L.

Dans le cadre de l'invention, on entend par « augmentation génique » une augmentation des ARNm codant la protéine d'intérêt.

Au sens de la présente invention, on entend par « augmenter l'expression génique et/ou protéique de collagène » une augmentation de l'expression protéique et/ou génique du collagène d'au moins 4%, préférentiellement d'au moins 20%, encore préférentiellement d'au moins 50%, avantageusement d'au moins 100%, et très avantageusement d'au moins 400%, en présence de l'extrait de *N. lappaceum* selon l'invention, par rapport à l'expression protéique et/ou génique de collagène détectée en l'absence de l'extrait.

Dans un mode de réalisation avantageux de l'invention, l'expression de collagène est l'expression protéique de collagène de type I et/ou V, encore préférentiellement de collagène de type I. Préférentiellement, ladite expression est mesurée dans des fibroblastes humains qualifiés de normaux, c'est-à-dire non pathologiques, encore préférentiellement en présence d'un extrait de *N. lappaceum* selon l'invention, avantageusement en présence de l'extrait 1a) ou de l'extrait 1e).

Préférentiellement, l'expression protéique de collagène de type I et/ou V est mesurée par technique immunohistochimique à l'aide d'un anticorps anti-collagène telle que décrite dans les conditions de l'exemple 2.

Dans un mode particulièrement avantageux de réalisation de l'invention, un extrait de feuilles de *N. lappaceum* est utilisé pour augmenter la fermeté de la peau et/ou des muqueuses en augmentant l'expression génique et/ou protéique, préférentiellement protéique, de collagène de type I et/ou V, préférentiellement de type I.

Dans un mode alternatif de réalisation de l'invention, un extrait de feuilles de *N. lappaceum* est utilisé pour augmenter la fermeté de la peau et/ou des muqueuses en diminuant l'expression génique et/ou protéique, préférentiellement protéique de CYR61.

On entend par « diminuer l'expression génique et/ou protéique de CYR61 » une diminution de l'expression génique et/ou protéique d'au moins 4%, préférentiellement d'au moins 15%, encore préférentiellement d'au moins 30% en présence de l'extrait de *N. lappaceum* selon l'invention, par rapport au niveau d'expression protéique et/ou génique de CYR61 détecté en l'absence de l'extrait. Dans un mode de réalisation avantageux, il s'agit d'une diminution de l'expression protéique de CYR61, avantageusement mesurée dans des fibroblastes humains normaux, c'est-à-dire non pathologiques, encore préférentiellement mesurée en présence d'un extrait de *N. lappaceum* selon l'invention, très avantageusement en présence de l'extrait 1a) ou 1e), dans les conditions décrites dans l'exemple 5.

En outre, on entend par « augmenter l'expression génique et/ou protéique de fibrilline » augmenter l'expression protéique et/ou génique de fibrilline d'au moins 30%, préférentiellement d'au moins 50%, encore préférentiellement d'au moins 100%, avantageusement d'au moins 150% et très avantageusement d'au moins 200% en présence de l'extrait de *N. lappaceum* selon l'invention par rapport au niveau d'expression protéique et/ou génique de fibrilline détecté en l'absence de l'extrait. Dans un mode de réalisation avantageux, il s'agit d'une augmentation de l'expression protéique de fibrilline-1, avantageusement mesurée dans des fibroblastes humains normaux, c'est-à-dire non pathologiques, encore préférentiellement mesurée en présence d'un extrait de *N. lappaceum,* avantageusement en présence de l'extrait 1a) ou de l'extrait 1e), dans les conditions décrites dans l'exemple 4.

Ainsi selon l'invention, l'extrait *de N. lappaceum* est utilisé pour augmenter l'élasticité de la peau et/ou des muqueuses, en augmentant l'expression de fibrilline, avantageusement de fibrilline-1.

Dans un mode de réalisation alternatif de l'invention, l'extrait de *N. lappaceum* est utilisé pour augmenter l'élasticité de la peau et/ou des muqueuses en augmentant l'expression génique et/ou protéique de LOX-L. On entend au sens de l'invention par « augmenter l'expression génique et/ou protéique de LOX-L » une augmentation d'au moins 2%, préférentiellement d'au moins 5%, encore préférentiellement d'au moins 10% et très préférentiellement d'au moins 15% de l'expression génique et/ou protéique de LOX-L en présence de l'extrait de *N. lappaceum* selon l'invention par rapport au niveau d'expression protéique et/ou génique de LOX-L détecté en l'absence de l'extrait. Dans un mode de réalisation avantageux, il s'agit d'une augmentation de l'expression protéique de LOX-L, avantageusement mesurée dans des fibroblastes humains normaux, c'est-à-dire non pathologiques, encore préférentiellement mesurée en présence d'un extrait de *N. lappaceum* selon l'invention, très avantageusement en présence de l'extrait 1a) ou 1e). Dans un autre mode de réalisation alternatif de l'invention, l'extrait augmente l'élasticité de la peau et/ou des muqueuses en augmentant l'expression génique et/ou protéique de fibuline-5. On entend au sens de l'invention par « augmenter l'expression génique et/ou protéique de fibuline-5 » une augmentation d'au moins 2%, préférentiellement d'au moins 5%, encore préférentiellement d'au moins 10% et très préférentiellement d'au moins 15% de l'expression génique et/ou protéique de fibulin-5 en présence de l'extrait de *N. lappaceum* selon l'invention, par rapport au niveau d'expression protéique et/ou génique de fibuline-5 détecté en l'absence de l'extrait. Dans un mode de réalisation avantageux, il s'agit d'une augmentation de l'expression protéique de fibuline-5, avantageusement mesurée dans des fibroblastes humains normaux, c'est-à-dire non pathologiques, encore préférentiellement mesurée en présence d'un extrait de *N. lappaceum* selon l'invention, très avantageusement en présence de l'extrait 1a) ou 1e).

Dans encore un autre mode de réalisation alternatif de l'invention, l'extrait augmente l'élasticité de la peau et/ou des muqueuses en augmentant l'expression génique et/ou protéique d'Emiline-1. On entend ainsi au sens de l'invention par « augmenter l'expression génique et/ou protéique d'Emiline-1 » une augmentation de l'expression protéique et/ou génique d'Emiline-1 d'au moins 2%, préférentiellement d'au moins 5%, encore préférentiellement d'au moins 10% et très préférentiellement d'au moins 15% en présence de l'extrait de *N. lappaceum* selon l'invention par rapport au niveau d'expression protéique et/ou génique d'Emiline-1 détecté en l'absence de l'extrait. Dans un mode de réalisation avantageux, il s'agit d'une augmentation de l'expression protéique d'Emiline-1, avantageusement mesurée dans des fibroblastes humains normaux, c'est-à-dire non pathologiques, encore préférentiellement mesurée en présence d'un extrait de *N. lappaceum* selon l'invention, très avantageusement en présence de l'extrait 1a) ou 1e).

Un objet de la présente invention est donc l'utilisation cosmétique d'un extrait de *N. lappaceum* pour augmenter l'élasticité de la peau et/ou des muqueuses, en augmentant l'expression génique et/ou protéique de fibrilline-1, de LOX-L, de fibuline-5 et/ou d'Emiline-1. Dans un mode avantageux de réalisation de l'invention, un extrait de feuilles et/ou de branches et/ou d'écorce et/ou de tige et/ou de graines, préférentiellement de feuilles, de *N. lappaceum* est utilisé pour augmenter l'expression génique et/ou protéique de fibrilline-1, de LOX-L, de fibuline-5 et/ou d'Emiline-1, préférentiellement de fibrilline-1, encore préférentiellement l'expression protéique de fibrilline-1, pour augmenter l'élasticité de la peau et/ou des muqueuses.

L'extrait selon la description peut être tout ou partie de la plante *N. lappaceum* choisi parmi l'écorce, les feuilles, les branches, la tige, le fruit entier, la pulpe du fruit, les graines, le péricarpe, la racine. Préférentiellement selon la description, l'extrait est un extrait de feuilles et/ou de graines et/ou de pulpe et/ou de branche. Selon l'invention, l'extrait est un extrait de feuilles. La plante entière ou la partie de la plante concernée est préférentiellement séchée et/ou broyée avant extraction.

On entend au sens de la présente invention par « pulpe » le fruit sans le péricarpe et sans la graine. On entend par « péricarpe » l'enveloppe du fruit, qualifiée aussi de coque. On entend encore par « écorce » l'écorce de l'arbre et/ou des branches. Ainsi, on entend par « branche » le bois et l'écorce. On entend par « graine » la graine sans la pulpe.

L'extrait peut être obtenu par différentes méthodes d'extraction connues de l'homme du métier, choisis parmi la macération, la décoction à chaud, par broyage dont le broyage aux ultrasons, à l'aide d'un mixeur, ou encore l'extrait peut être obtenu par extraction dans l'eau en conditions subcritiques. Préférentiellement, l'extraction est réalisée par macération. Dans un mode particulièrement avantageux de réalisation, l'extraction est réalisée dans l'eau en conditions subcritiques. Avantageusement, l'extraction n'est pas réalisée en conditions supercritiques (CO₂). L'extraction pourra être réalisée à une température allant de 4°C à 300°C, y inclus la température ambiante, c'est-à-dire une température de 20°C. Dans un mode préférentiel de réalisation de l'invention, l'extraction sera réalisée à une température de 60°C à 90°C, préférentiellement de 70°C à 85°C, encore préférentiellement à une température de 80°C.

Dans un mode alternatif de réalisation de l'invention, l'extraction sera conduite à une température de 4°C à 25°C, encore préférentiellement de 4°C à 20°C, encore avantageusement à température ambiante, c'est-à-dire à 20°C.

Dans encore un autre mode alternatif de réalisation de l'invention, l'extraction sera réalisée dans l'eau en conditions subcritiques, à une température allant de 100°C à 374°C, avantageusement de 120°C à 250°C, encore avantageusement à 120°C. L'extraction peut être réalisée à une température donnée unique ou à des températures successives croissantes. Dans un mode de réalisation avantageux de l'invention, l'extraction sera réalisée séquentiellement à trois températures croissantes de 120°C, 140°C et 160°C.

On entend par extraction en « conditions subcritiques » une extraction en présence d'eau, dans des conditions de température supérieures à 100°C et de pression inférieure à 221 bars, telle que l'eau reste à l'état liquide mais possède une viscosité et une tension de surface inférieures à celle de l'eau à température ambiante, augmentant sa constante diélectrique.

Ainsi, la pression d'extraction sera comprise entre 150 bars et 250 bars, préférentiellement entre 200 et 221 bars, avantageusement dans un autoclave d'extraction sous pression.

L'extraction peut être conduite durant une période de 30 minutes à 24 heures, préférentiellement de 30 minutes à 12 heures, encore préférentiellement durant une période de 1 heure à 5 heures, et encore avantageusement durant une période de 1 heure à 2 heures. Très avantageusement, l'extraction sera conduite durant une période de 1 heure.

L'extrait selon l'invention pourra être obtenu par extraction dans un solvant ou mélange de solvant de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol et/ou butylène glycol et/ou autres glycols tel que xylitol et/ou propanediol, etc.) de 99/1 à 1/99 (p/p), avantageusement dans l'eau comme unique solvant. Ainsi, dans un mode de réalisation particulier, l'extrait est un extrait de feuille préparé dans l'eau comme unique solvant.

De façon particulière, l'extrait est obtenu par extraction aqueuse. Au sens de la présente invention, on entend par « extrait obtenu par extraction aqueuse » tout extrait obtenu par extraction avec une solution aqueuse contenant plus de 60% en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de glycol et de façon particulière ne contenant pas d'alcool, plus particulièrement ne contenant que de l'eau.

Dans un mode de réalisation alternatif, l'extrait est obtenu par extraction dans un mélange de propanediol et d'eau en proportion respective (80, 20 ; v/v).

Dans un autre mode alternatif de réalisation de l'invention, l'extraction pourra par ailleurs être réalisée en présence d'un surfactant non ionique, préférentiellement choisi parmi du lauryl glucoside commercialisé sous le nom de Plantacare^{®} 1200UP par BASF ou encore du caprylyl/capryl glucoside (Plantacare^{®} 810 UP), préférentiellement du caprylyl/capryl glucoside (Plantacare^{®} 810 UP). La concentration en poids du surfactant non ionique pourra être comprise entre 0,5% et 5%, avantageusement entre 0,5 et 1%, encore avantageusement elle sera de 1% en poids par rapport au poids total de l'extrait.

L'extrait peut être obtenu par extraction d'une quantité de 0,1% à 10 % en poids de matière fraîche ou sèche, préférentiellement sèche, d'au moins une partie de la plante de *N. lappaceum* par rapport au poids total du mélange solvant/plante (p/p). Préférentiellement, l'extrait est obtenu par extraction d'une quantité de 1% à 10% en poids, avantageusement de 5% à 10%, encore avantageusement de 10% en poids de matière sèche d'au moins une partie de la plante, par rapport au poids total du mélange constitué du solvant, préférentiellement l'eau, et de la plante (p/p). Dans un mode de réalisation particulier de l'invention, l'extrait sera obtenu à partir d'une quantité de 10% en poids d'au moins une partie de la plante par rapport au poids total du mélange solvant/plante (p/p) puis concentré à 20%. Préférentiellement alors, selon la description, la partie de plante sera les graines.

Ainsi, dans un mode avantageux de réalisation de l'invention, l'extrait est obtenu par macération dans l'eau comme unique solvant, d'une quantité de 10% en poids de feuilles séchées de la plante *N. lappaceum* par rapport au poids total de feuilles et d'eau, à une température de 80°C, durant une période de 1 heure, dans les conditions décrites dans l'exemple 1a). L'extrait brut obtenu est ensuite décanté, centrifugé puis filtré. L'extrait obtenu pourra être éventuellement être séché et se présentera sous forme de poudre.

Dans un autre mode de réalisation, l'extrait est obtenu par macération à partir d'une quantité de 5% en poids de feuilles séchées de la plante *N. lappaceum* par rapport au poids total de feuilles et d'eau, à une température de 80°C, durant une période de 1 heure, dans les conditions décrites dans l'exemple 1b). L'extrait brut obtenu est ensuite décanté, centrifugé puis filtré.

Dans encore un autre mode de réalisation de la description, l'extrait est obtenu par macération à partir d'une quantité de 10% en poids de branche séchée de la plante *N. lappaceum* par rapport au poids total de branche et d'eau, à température ambiante, c'est-à-dire à une température de 20°C, durant une période de 2 heures, dans les conditions décrites dans l'exemple 1c). Avantageusement dans ce cas, l'extraction est effectuée en présence d'une concentration en poids de 1% de caprylyl/capryl glucoside (Plantacare^{®} 810 UP). L'extrait brut obtenu est ensuite décanté, centrifugé puis filtré.

Dans un 4^{ème} mode de réalisation de l'invention, l'extraction est réalisée par macération à partir d'une quantité de 10% en poids de feuilles séchée de *N. lappaceum* dans un mélange propanediol/eau (80, 20 ; v/v) à une température de 80°C durant une période de 1 heure, dans les conditions décrites dans l'exemple 1d). L'extrait brut obtenu est ensuite décanté, centrifugé puis filtré.

Dans un 5^{ème} mode de réalisation de l'invention, l'extraction est réalisée par extraction dans l'eau en conditions subcritiques, d'une quantité de 10% en poids de feuilles séchée de *N. lappaceum* dans un autoclave d'extraction sous pression, à une température de 250°C, sous pression de 250 bars dans les conditions décrites dans l'exemple 1e). L'extrait brut obtenu est ensuite décanté, centrifugé puis filtré. L'extrait obtenu pourra être éventuellement être séché.

Dans un 6^{ème} mode de réalisation de la description, l'extraction sera réalisée par macération d'une quantité de 10% en poids de la pulpe du fruit par rapport au poids total de pulpe et de l'eau comme unique solvant, à une température de 80°C, durant une période de 1 heure, dans les conditions décrites dans l'exemple 1f). Alternativement, l'extraction de pulpe pourra être réalisée à partir d'une quantité de 10% en poids de pulpe, dans un mélange propanediol/eau (80, 20 ; v/v) à une température de 80°C durant une période de 1 heure. Dans encore un autre mode de réalisation alternatif, l'extraction de pulpe pourra être effectuée par extraction dans l'eau en conditions subcritiques. L'extrait brut obtenu est ensuite décanté, centrifugé puis filtré.

Dans un 7^{ème} mode de réalisation de la description, l'extraction sera réalisée par macération d'une quantité de 10% de graines en poids par rapport au poids total des graines et de l'eau comme unique solvant, à température ambiante, c'est-à-dire à 20°C, durant une période de 2 heures, dans les conditions décrites dans l'exemple 1g). Alternativement, l'extraction de graines pourra être réalisée à partir d'une quantité de 10% en poids de graines, dans un mélange propanediol/eau (80, 20 ; v/v) à une température de 80°C durant une période de 1 heure. Dans encore un autre mode de réalisation alternatif, l'extraction de graines pourra être effectuée par extraction dans l'eau en conditions subcritiques. L'extrait brut obtenu est ensuite décanté, centrifugé puis filtré.

L'extrait obtenu et utilisé selon l'invention peut être ensuite centrifugé et/ou filtré et/ou distillé pour récupérer la fraction soluble, préférentiellement la fraction hydrosoluble. Préférentiellement, le surnageant obtenu est ensuite filtré, avantageusement à un seuil de coupure de 0,45 µm. Des étapes supplémentaires de décoloration et/ou désodorisation peuvent être effectuées sur l'extrait à n'importe quel stade de l'extraction et selon les techniques connues par l'Homme du métier. De façon particulière, l'extrait pourra être décoloré au charbon actif.

L'extrait peut être ensuite concentré par évaporation du solvant ou séché par exemple par lyophilisation ou par atomisation en présence de maltodextrines. L'extrait se présentera alors sous forme de poudre.

Ainsi dans un mode de réalisation préférentiel de l'invention, l'extrait obtenu sera atomisé en présence d'une concentration en poids de maltodextrines comprise entre 20% et 90%, préférentiellement entre 40 et 80%, encore préférentiellement de 70 à 80% par rapport au poids total de la poudre obtenue.

Dans un mode particulier de réalisation de l'invention, notamment pour son utilisation en dermatologie, l'extrait de *N. lappaceum* obtenu est stérilisé.

L'extrait peut être utilisé seul sous forme d'ingrédient cosmétique ou dermatologique, ou dans une composition cosmétique ou dermatologique, comprenant au moins un excipient cosmétiquement ou dermatologiquement acceptable.

Lorsqu'il est utilisé seul sous forme d'ingrédient cosmétique ou dermatologique, il est préférentiellement solubilisé dans et/ou dilué dans un solvant notamment polaire, tel que l'eau, un alcool, un polyol, un glycol, tel que le pentylène glycol et/ou le butylène glycol et/ou l'hexylène glycol et/ou le caprylyl glycol, ou un de leurs mélanges, préférentiellement un mélange hydroglycolique, encore préférentiellement contenant un glycol choisi parmi l'hexylène glycol, le caprylyl glycol et leurs mélanges. Avantageusement l'extrait obtenu est dilué et/ou soluble dans une solution aqueuse contenant de l'hexylène glycol, en particulier contenant entre 0,1 et 10 % en poids de l'hexylène glycol, préférentiellement entre 0,5 et 5 % en poids d'hexylène glycol, par rapport au poids total la solution aqueuse. Avantageusement l'extrait obtenu est dilué et/ou soluble dans une solution aqueuse contenant du caprylyl glycol, en particulier contenant entre 0,01 et 5 % en poids de caprylyl glycol, préférentiellement entre 0,1 et 1 % en poids de caprylyl glycol, par rapport au poids total la solution aqueuse. De façon particulière, la solution aqueuse dans laquelle est solubilisée l'extrait de *N. lappaceum* selon l'invention comprend de la gomme xanthane, en particulier entre 0,01 et 5 % en poids de gomme xanthane par rapport au poids total la solution aqueuse, plus particulièrement entre 0,1 et 1 % en poids de gomme xanthane par rapport au poids total de la solution aqueuse.

De façon avantageuse, la solution dans laquelle est solubilisé l'extrait de *N. lappaceum* selon l'invention comprend de l'hexylène glycol, du caprylyl glycol et de la gomme de xanthane.

Dans un mode alternatif de réalisation de l'invention, l'extrait sera solubilisé dans une solution aqueuse comprenant de la glycérine à une concentration en poids par rapport au poids total de l'ingrédient cosmétique de 50% à 85%, avantageusement de 60% à 80%, encore avantageusement de 79%, du biopropanediol à une concentration en poids par rapport au poids total de l'ingrédient cosmétique de 5% à 20%, avantageusement de 10%, et de l'eau.

L'extrait peut aussi être présent dans une composition cosmétique comprenant en outre au moins un excipient cosmétiquement acceptable. On entend par « acceptable » un excipient cosmétique ou non irritant pour la peau, n'induisant pas de réponse allergique, et stable sur le plan chimique.

Un objet de la présente invention concerne donc l'utilisation de l'extrait de la plante *N. lappaceum* selon l'invention dans une composition cosmétique pour augmenter la fermeté et/ou l'élasticité de la peau et/ou des muqueuses, en augmentant l'expression génique et/ou protéique de collagène de type I et/ou V, préférentiellement de collagène type I, de LOX-L, de fibuline-5, d'Emiline-1 et/ou de fibrilline, préférentiellement de fibrilline-1, et/ou en diminuant l'expression de CYR61, au niveau de la peau et/ou des muqueuses.

La composition cosmétique comprenant l'extrait de *N. lappaceum* selon l'invention pourra être appliquée, préférentiellement par voie topique, sur tout ou partie du corps et/ou du visage ou une augmentation de fermeté et/ou d'élasticité est souhaitée, préférentiellement les jambes, les cuisses, les bras, le ventre, le décolleté, le cou, tout ou partie du visage, préférentiellement les joues, le front, le menton, les lèvres, le contour des lèvres, le contour des yeux, la zone dite « en T » du visage.

Dans un mode de réalisation de l'invention, l'extrait est présent dans la composition cosmétique ou dermatologique à une concentration de 1.10⁻⁴ % à 10%, préférentiellement de 1.10⁻⁴ % à 5 %, et encore préférentiellement de 1.10⁻³ % à 3% en poids, par rapport au poids total de la composition. Le ou les excipients pourront être choisis parmi des agents tensioactifs et/ou émulsifiants, des conservateurs, des agents tampons, des agents chélatants, des dénaturants, des agents opacifiants, des ajusteurs de pH, des agents réducteurs, des agents stabilisants, des épaississants, des gélifiants, des polymères filmogènes, des charges, des agents matifiants, des agents de brillance, des pigments, des colorants, des parfums, et leurs mélanges. Le CTFA (Cosmetic Ingrédient Handbook, Second Edition (1992)) décrit différents excipients cosmétiques adaptés à une utilisation dans la présente invention.

Avantageusement, le ou les excipients sont choisis dans le groupe comprenant les polyglycérols, les esters, les polymères et dérivés de cellulose, les dérivés de lanoline, les phospholipides, les lactoferrines, les lactoperoxidases, les stabilisants à base de sucrose, la vitamine E et ses dérivés, les gommes de xanthane, les cires naturelles et synthétiques, les huiles végétales, les triglycérides, les insaponifiables, les phytostérols, les silicones, les hydrolysats de protéines, les betaines, les aminoxides, les extraits de plantes, les esters de saccharose, les dioxydes de titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparabéne, l'éthylparabène, le propylparabène, le butylparabène, le butylène glycol, le caprylyl glycol, les tocophérols naturels, la glycérine, le dihydroxycetyl sodium phosphate, l'isopropyl hydroxycétyl éther, le glycol stéarate, la triisononanoine, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les triglycérides caprique/caprylique, le cétéaryl octanoate, le dibutyl adipate, l'huile de pépins de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, la cire d'abeille, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée, et leurs mélanges.

La composition cosmétique selon l'invention peut être choisie parmi une solution, aqueuse ou huileuse, une crème ou un gel aqueux ou un gel huileux, notamment un gel douche, un lait, une émulsion, une microémulsion ou une nanoémulsion, notamment huile-dans-eau ou eau-dans huile ou multiple ou siliconée, un masque, un sérum, une lotion, un savon liquide, un pain dermatologique, une pommade, une mousse, un patch, un produit anhydre, de préférence liquide, pâteux ou solide, par exemple sous forme de poudres de maquillage, de bâtonnet ou de stick, notamment sous forme de rouge à lèvre. De façon avantageuse, il s'agit d'une crème ou d'un sérum.

La composition utilisée selon l'invention peut en outre contenir des ingrédients actifs cosmétiques conduisant à un effet complémentaire ou synergique tels que des actifs anti-âges. On citera parmi ceux-ci des actifs stimulant la synthèse des macromolécules du derme ou empêchant leur dégradation, des agents stimulant la prolifération des kératinocytes, des agents apaisants, hydratants, ou encore des agents actifs sur la régulation de la taille des pores et/ou leur ouverture. Parmi les actifs anti-âges, on citera :
- un agent stimulant la synthèse de fibronectine, en particulier un extrait de maïs, un tel extrait étant notamment commercialisé par BASF Beauty Care Solutions France sous le nom Deliner^{™} et le palmitoyl pentapeptide commercialisé par la société SEDERMA sous la dénomination commerciale Matrixil^{®} ;
- un agent stimulant la formation des fibres de collagène comme un extrait d*'Origanum majorana* commercialisé sous le nom Dermagenist^{™} par BASF Beauty Care Solutions France.
- un agent stimulant l'expression du perlécan et du dystroglycan dans la matrice extracellulaire et/ou dans la membrane basale épithéliale comme par exemple un extrait de *Polygonum bistorta* commercialisé sous le nom Perlaura^{™} par BASF Beauty Care Solutions France.
- un agent de protection du facteur de croissance des fibroblastes (FGF2) de la matrice extracellulaire contre sa dégradation et/ou sa dénaturation, notamment un extrait d*'Hibiscus abelmoscus* tel que décrit dans la demande de brevet au nom de la BASF Beauty Care Solutions France déposée sous le numéro FR0654316 et commercialisé par BASF Beauty Care Solutions France sous le nom Linefactor ^{™} et/ou un agent de stimulation de croissance des fibroblastes par exemple un extrait de soja fermenté contenant des peptides, connu sous le nom de Phytokine^{™} commercialisé par BASF Beauty Care Solutions France et également décrit dans la demande de brevet EP1119344B1 (Laboratoires Expanscience), et préférentiellement une combinaison de ces deux extraits ;
- un agent stimulant la synthèse de laminine, en particulier un extrait de malt modifié par biotechnologie, un tel extrait étant notamment commercialisé par BASF Beauty Care Solutions France sous le nom Basaline^{™} ;
- un agent stimulant l'expression et/ou l'activité de la Hyaluronane synthase 2 (HAS2) tels que les extraits végétaux décrits dans la demande de brevet FR2893252 et en particulier un extrait aqueux de Galanga (*Alpinia galanga*) et commercialisé par BASF Beauty Care Solutions France sous le nom Hyalufix^{™};
- un agent stimulant la synthèse de lysyl oxydase like (LOXL) tel qu'un extrait de *Geophila cordifolia* et ceux décrits dans la demande de brevet FR2855968, et en particulier un extrait d'aneth et commercialisé par BASF Beauty Care Solutions France sous le nom Lys'lastine ^{™};
- un agent stimulant la synthèse d'ATP intracellulaire, notamment un extrait d'algue *Laminaria digitata ;*
- un actif stimulant la synthèse des glycosaminoglycanes, tel qu'un produit de fermentation du lait
- un actif stimulateur de collagène tel que le rétinol et/ou la vitamine C ;
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, l'extrait de feuilles d*'Argania spinosa* commercialisé par BASF Beauty Care solutions France SAS sous le nom Arganyl^{™} ; le lycopène ; les isoflavones, la quercetine, le kaempferol, l'apigénine.
- un agent de regonflement notamment les sphères de comblement d'acide hyaluronique commercialisé par BASF Beauty Care Solutions France (Hyaluronic Filling Spheres ^{™}).
- un agent pour augmenter l'expression de LOX pour augmenter l'architecture de l'épiderme comme par exemple un extrait de *Cichorium intybus* commercialisé sous le nom de LOX-AGE^{™} par BASF Beauty Care Solutions France.
- un agent pour augmenter la déglycation du collagène et/ou augmenter l'expression du collagène de type I tel qu'une combinaison d'un extrait de feuilles de *Salvia miltiorrhiza* et de niacinamide commercialisé par BASF Beauty Care Solutions France sous le nom CollRepair^{™}.
- un agent stimulant la synthèse de lumican et de collagène tel qu'un tétrapeptide synthétique acetyl Gln Asp Val His commercialisé par BASF Beauty Care Solutions France sous le nom Dermican^{™} et décrit dans la demande de brevet WO2005120554.
- un agent de protection et de stimulation de l'élastine, du collagène comme l'extrait de feuilles de *Manilkara multinervis* commercialisé par BASF Beauty Care Solutions France sous le nom Elestan^{™} et l'extrait de racine de *Eperuafalcata* commercialisé par BASF Beauty Care Solutions France sous le nom Eperuline^{™}.
- un agent anti-taches pigmentaire notamment par inhibition de synthèse de mélanine tel que le complexe synergique d'extrait de *Pisum sativum* et de sucrose dilaurate commercialisé par BASF Beauty Care Solutions France sous le nom Actiwhite^{™}, ou l'acide hydroxyphenoxy propionique commercialisé par BASF Beauty Care Solutions France sous le nom Radianskin^{™}.
- un agent stimulant la fermeté et l'élasticité de la peau tel qu'un extrait de *Khaya senegalensis,* commercialisé par la demanderesse sous le nom de Collalift^{®}18.

Les agents stimulant la prolifération des kératinocytes, préférentiellement utilisables dans la composition selon l'invention, comprennent notamment les rétinoïdes tels que le rétinol et ses esters, dont le palmitate de rétinyle, et le phloroglucinol. Les agents stimulant la différenciation des kératinocytes comprennent par exemple les minéraux tels que le calcium et les lignanes tels que le sécoisolaricirésinol, ainsi que l'extrait d*'Achillea millefollium* commercialisé sous le nom de Neurobiox^{™} par BASF Beauty Care Solutions France.

Comme agents apaisants préférentiellement utilisables dans la composition selon l'invention, on peut citer : les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de *Pueraria lobata* commercialisé sous le nom Inhipase^{®} par BASF Beauty Care Solutions France SAS, les extraits de *Theobroma cacao.*

Parmi les agents actifs sur la régulation de la taille des pores et/ou leur ouverture et/ou sur la production de sébum, on citera à titre d'exemple un extrait de *Cichorium intybus* commercialisé sous le nom de LOX-AGE^{™} par BASF Beauty Care Solutions France, ou de la sarcosine synthétique commercialisée sous le nom de Mat-XS^{™} Clinical et/ou un extrait *d'Orthosiphon stamineus* tel que décrit dans la demande de brevet WO2010063674 au nom de BASF Beauty Care Solutions France et commercialisé sous le nom MAT XS^{™} Bright.

Comme agents hydratants préférentiellement utilisables dans la composition selon l'invention, on peut citer : une combinaison de pullulan, de hyaluronate de sodium et d'alginate de sodium tel que celle commercialisée par BASF Beauty Care Solutions France sous le nom PatcH₂O^{™}.

Un autre objet de la description concerne encore un procédé de soin cosmétique comprenant l'application, par voie topique ou orale, préférentiellement par voie topique, de l'extrait selon l'invention ou d'une composition cosmétique le comprenant pour augmenter la fermeté et/ou l'élasticité de la peau et/ou des muqueuses.

Ainsi, le procédé de soin cosmétique selon la description est pour augmenter l'expression génique et/ou protéique, de collagène de type I et/ou V, préférentiellement de collagène de type I, et/ou diminuer l'expression de CYR61, au niveau de la peau et/ou des muqueuses pour augmenter la fermeté de la peau et/ou des muqueuses.

Un autre objet de la description concerne, un procédé de soin cosmétique qui comprend l'application par voie topique ou orale, préférentiellement par voie topique, de l'extrait de *N. lappaceum* selon l'invention ou d'une composition cosmétique le comprenant pour augmenter l'élasticité de la peau et/ou des muqueuses, en augmentant l'expression génique et/ou protéique, de fibrilline, préférentiellement de fibrilline-1, de LOX-L, de fibuline-5, et/ou d'Emiline-1, encore préférentiellement de fibrilline-1, et avantageusement protéique, au niveau de la peau et/ou des muqueuses.

Dans un mode de réalisation de la description, le procédé comprend l'application par voie topique sur tout ou partie de peau du corps et/ou du visage où une augmentation de la fermeté et/ou de l'élasticité est souhaitée, préférentiellement les jambes, les cuisses, les bras, le ventre, le décolleté, le cou, tout ou partie du visage, préférentiellement les joues, le front, le menton, les lèvres, le contour des lèvres, le contour des yeux, la zone dite « en T » du visage, de l'extrait selon l'invention ou d'une composition cosmétique le comprenant.

Enfin, un autre objet de la présente invention concerne l'extrait de *N. lappaceum* pour son utilisation, par voie topique ou orale et préférentiellement par voie topique, seul ou dans une composition pharmaceutique, préférentiellement dermatologique le comprenant, dans la prévention et/ou le traitement de pathologies impliquant une perte d'expression protéique et/ou génique de collagène, préférentiellement de collagène de type I et/ou une perte de fermeté pathologique de la peau et/ou des muqueuses telles que couperose, télangiectasies.

Enfin, un autre objet de la présente invention concerne l'extrait de *N. lappaceum* pour son utilisation, par voie topique ou orale et préférentiellement par voie topique, seul ou dans une composition pharmaceutique, préférentiellement dermatologique le comprenant, dans la prévention et/ou le traitement de pathologies impliquant une perte d'expression protéique et/ou génique de fibrilline préférentiellement de fibrilline de type I et/ou une perte de élasticité pathologique de la peau et/ou des muqueuses telles que élastose solaire, maladie cutix laxa et/ou vergetures.

Dans un mode de réalisation de l'invention, l'extrait est compris dans la composition dermatologique ou pharmaceutique comprenant par ailleurs au moins un excipient dermatologiquement ou pharmaceutiquement acceptable, à une concentration de 1.10⁻⁴ % à 10%, préférentiellement de 1.10⁻⁴ % à 5%, et encore préférentiellement de 1.10⁻³ % à 3% en poids, par rapport au poids total de la composition.

Des exemples faisant référence à la description de l'invention sont présentés ci-après. Ces exemples sont donnés à titre d'illustration et ne sauraient limiter en aucun cas la portée de l'invention. Chacun des exemples a une portée générale. Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention.

### EXEMPLES :

### Exemple 1 : Préparation de différents extraits de N. lappaceum selon l'invention

Exemple 1a) L'extrait a été obtenu par macération dans l'eau comme unique solvant, d'une quantité de 10% en poids de feuilles séchées de la plante *N. lappaceum* par rapport au poids total de feuilles et d'eau comme unique solvant, à une température de 80°C, durant une période de 1 heure. L'extrait brut a été décanté, centrifugé puis filtré. Cet extrait pourra être ensuite éventuellement séché.

Exemple 1b) L'extrait a été obtenu par macération à partir d'une quantité de 5% en poids de feuilles de la plante *N. lappaceum* par rapport au poids total de feuilles séchées et d'eau comme unique solvant, à une température de 80°C, durant une période de 1 heure. L'extrait brut a été centrifugé puis filtré.

Exemple comparatif 1c) L'extrait a été obtenu par macération à partir d'une quantité de 10% en poids de branche séchées de la plante *N. lappaceum* par rapport au poids total des branches et d'eau comme unique solvant, à température ambiante, c'est-à-dire à une température de 20°C, durant une période de 2 heures en présence d'une concentration en poids de 1% de caprylyl/capryl glucoside (Plantacare^{®} 810 UP). L'extrait brut a été décanté, centrifugé puis filtré.

Les extraits obtenus dans les exemples 1a) à 1c) ont ensuite été concentrés par évaporation du solvant et séchés par atomisation en présence de maltodextrines. Ces extraits se présentent sous forme de poudre.

Exemple 1d) L'extraction a été réalisée par macération à partir d'une quantité de 10 % en poids de feuilles séchées de *N. lappaceum* dans un mélange propanediol/eau (80, 20 ; v/v) à une température de 80°C durant une période de 1 heure. L'extrait brut a été décanté, centrifugé puis filtré.

Exemple 1e) L'extraction a été réalisée à partir d'une quantité de 10 % en poids de feuilles séchées de *N. lappaceum* dans l'eau en conditions subcritiques, dans un autoclave d'extraction sous pression, à une température de 250°C, sous pression de 250 bars. L'extrait brut a été décanté, centrifugé puis filtré.

Exemple comparatif 1f) L'extraction a été réalisée par macération dans l'eau comme unique solvant à partir d'une quantité de 10% en poids de pulpe de fruit de *N. lappaceum* par rapport au poids total de la pulpe et de l'eau, à une température de 80°C durant une période de 1 heure. L'extrait brut a été décanté, centrifugé puis filtré.

Exemple comparatif 1g) L'extraction a été réalisée par macération dans l'eau comme unique solvant d'une quantité de 10% de graines en poids par rapport au poids total des graines et de l'eau, à température ambiante, c'est-à-dire à 20°C, durant une période de 2 heures. L'extrait brut a été décanté, centrifugé puis filtré.

Exemple comparatif 1h) L'extrait a été obtenu par macération à partir d'une quantité de 10% en poids de branche séchée de la plante *N. lappaceum* par rapport au poids total de branche et d'eau comme unique solvant, à une température de 80°C, durant une période de 1 heure. L'extrait brut a été décanté, centrifugé puis filtré.

### Exemple 2 : Augmentation de l'expression de collagène de type I en présence de différents extraits de N. lappaceum selon l'invention.

*Protocole* : des fibroblastes humains dits « normaux », c'est-à-dire ne présentant pas de pathologie, issus d'une donneuse saine de 34 ans ont été cultivés dans un milieu défini (FGM) durant une période de 48 heures en présence de 2 concentrations finales différentes de différents extraits de *N. lappaceum* puis le milieu cellulaire a été éliminé. Le même milieu de culture sans ajout d'extrait selon l'invention a été utilisé comme témoin (Contrôle). Le tapis cellulaire obtenu a été lysé avec une solution d'hydroxyde d'ammonium.

Le dosage du collagène de type I a été effectué à partir du lysat obtenu avec un anticorps anti-collagène I, utilisé à 500 ng/mL dans une solution tampon (PBS). Après une période de 60 minutes, un anticorps secondaire utilisé à 40 ng/mL a été appliqué durant une période de 60 minutes. Après lavage, une solution de révélation a été ajoutée et la fluorescence a été mesurée (ENVision, PerkinElmer). Les résultats de fluorescence ont été normalisés par rapport à la fluorescence obtenue avec le même milieu cellulaire en l'absence de l'extrait de *N. lappaceum* (Contrôle) et ont été rapportés à la quantité d'ADN obtenue dans chaque condition. Les résultats présentés correspondent à la moyenne de 6 essais (n=6). (ET : Ecart-type).

### Résultats :

**Tableau 1**

| | MOY | ET |
|---|---|---|
| Contrôle | 100 | 14 |
| Extrait de feuilles de *N. lappaceum à* 1 % (p/v milieu préparé selon l'ex. 1a) | 290 | 72 |
| Extrait de graines de *N. lappaceum à* 10 % (p/v milieu) préparé selon l'ex. 1g) | 103 | 5 |
| Extrait de branche de *N. lappaceum à* 10 % (p/v milieu) préparé selon l'ex. 1h) | 94 | 10 |
| Extrait de pulpe de *N. lappaceum à* 10% (p/v milieu) selon l'ex. 1f) | 95 | 8 |

*Conclusion :* l'extrait de feuilles de *N. lappaceum* a augmenté l'expression protéique du collagène de type I d'au moins 104% et jusqu'à 686% dans les fibroblastes analysés.

### Exemple 3) Augmentation de l'expression de collagène de type V en présence de différents extraits de N. lappaceum

*Protocole* : des fibroblastes humains dits « normaux », c'est-à-dire ne présentant pas de pathologie, issus d'une donneuse saine de 34 ans ont été cultivés dans un milieu défini (FGM) durant une période de 48 heures en présence de concentrations finales différentes de différents extraits de *N. lappaceum* puis le milieu cellulaire éliminé. Le même milieu de culture sans ajout d'extrait selon l'invention a été utilisé comme témoin (Contrôle). Le tapis cellulaire obtenu a été lysé avec une solution d'hydroxyde d'ammonium, puis le dosage du collagène de type V a été effectué avec un anticorps anti-collagène V, dilué au 1/4000 dans une solution tampon (PBS). Après une période de 60 minutes, un anticorps secondaire dilué au 1/25000 a été appliqué durant une période de 60 minutes. Après lavage, une solution de révélation a été ajoutée et la fluorescence a été mesurée (ENVision, PerkinElmer). Les résultats de fluorescence ont été normalisés par rapport à la fluorescence obtenue avec le même milieu cellulaire en l'absence de l'extrait de *N. lappaceum* (Contrôle) et ont été rapportés à la quantité d'ADN obtenue dans chaque condition. Les résultats présentés correspondent à la moyenne de 3 essais (n=3) (ET : Ecart-type).

### Résultats :

**Tableau 2**

| | MOY | ET |
|---|---|---|
| Contrôle | 100 | 5 |
| Extrait de feuilles de *N. lappaceum* 1 % (p/v milieu) préparé selon l'ex. 1a) | 231 | 39 |
| Extrait de branche de *N. lappaceum* 0,1 % (p/v milieu) préparé selon l'ex. 1c) | 176 | 24 |
| Extrait de graines de *N. lappaceum* 1 % (p/v milieu) préparé selon l'ex. 1g) | 134 | 25 |
| Extrait de pulpe de *N. lappaceum* 0,5 % (p/v milieu) préparé selon l'exemple 1f) | 131 | 10 |

| | | |
|---|---|---|
| *Conclusion :* les extraits de *N. lappaceum* ont augmenté l'expression protéique du collagène de type V d'au moins 4% et jusqu'à au moins 400% dans les fibroblastes normaux analysés, démontrant leurs propriétés d'augmentation de la fermeté de la peau et des muqueuses. | | |

### Exemple 4 : Augmentation de l'expression de fibrilline-1 en présence de différents extraits de N. lappaceum

*Protocole:* des fibroblastes humains dits normaux, c'est-à-dire ne présentant pas de pathologie, issus d'une donneuse saine de 34 ans ont été cultivés dans un milieu défini (FGM) durant une période de 48 heures en présence de 2 concentrations finales différentes de l'extrait de *N. lappaceum* puis le milieu cellulaire a été éliminé. Le même milieu de culture sans ajout d'extrait a été utilisé comme témoin (Contrôle). Les cellules ont ensuite été récoltées puis lysées avec un tampon de lyse spécifique afin de procéder à l'immuno-localisation (Western Blot). La concentration protéique a été déterminé par la méthode BCA. Les protéines ont été identifiées par électrophorèse capillaire (ProteinSimple, USA) à l'aide d'un anticorps primaire anti-fibrilline et immunolocalisées à l'aide d'un anticorps secondaire conjugué couplé à la peroxidase. Les résultats ont été quantifiés à l'aide du logiciel Compass Software (version 2.7.1 (ProteinSimple)).

### Résultats:

**Tableau 3**

| | MOY | ET |
|---|---|---|
| Contrôle | 100 | 9 |
| Extrait de feuilles de *N. lappaceum* 1 % (p/v milieu) préparé selon l'exemple 1a) | 239 | 56 |
| Extrait de branches *N. lappaceum* 0,1 % (p/v milieu) préparé selon l'exemple 1c) | 148 | 1 |
| Extrait de graines *N. lappaceum* 1 % (p/v milieu) préparé selon l'exemple 1g) | 272 | 95 |
| Extrait de pulpe de *N. lappaceum* 0,5 % (p/v milieu) préparé selon l'exemple 1f) | 166 | 18 |

| | | |
|---|---|---|
| *Conclusion* : les résultats ont montré une augmentation d'au moins 30% et jusqu'à au moins 200% de l'expression protéique de fibrilline-1 dans les fibroblastes humains normaux, montrant sa capacité d'augmentation de l'élasticité de la peau et/ou des muqueuses. | | |

### Exemple 5 : Diminution de l'expression protéique de CYR61 en présence d'un extrait de N. lappaceum

*Protocole* : des fibroblastes humains dits « normaux », c'est-à-dire ne présentant pas de pathologie, issus d'une donneuse saine de 34 ans ont été cultivés dans un milieu défini (FGM) durant une période de 48 heures en présence de concentrations finales différentes d'extraits de *N. lappaceum* puis le milieu cellulaire éliminé. Le même milieu de culture sans ajout d'extrait selon l'invention a été utilisé comme témoin (Contrôle). Les surnageants de cultures ont ensuite été récupérés pour analyse. La concentration protéique a été déterminée par dosage BCA (BiCinchoninic acid Assay). L'expression protéique a été mesurée par Western Blot (n =4) (SallySue^{®}, ProteinSimple^{®}). La protéine d'intérêt CYR61 a été détectée par électrophorèse capillaire (Anticorps AbCam (ab24448)e dilué au 1/50) puis révélé avec un anticorps secondaire couplé à la Peroxydase de raifort et un substrat chémiluminescent. Le signal chémiluminescent a ensuite été détecté et quantifié (Compass^{®} version 2.7.1 (ProteinSimple^{®})).

### Résultats :

Les résultats sont exprimés en pourcentages relatifs par rapport au niveau d'expression protéique de CYR61 dans les surnageants de fibroblastes non-traités (Contrôle).

**Tableau 4**

| | MOY | ET |
|---|---|---|
| Contrôle | 100 | 7,8 |
| Extrait séché de feuilles de *N. lappaceum* préparé selon l'exemple 1a) (4×10⁻³ p/v milieu) | 79,4 | 8,2 |
| Extrait séché de feuilles de *N. lappaceum* préparé selon l'exemple 1a) (8×10⁻³ p/v milieu) | 52,4 | 7,9 |

*Conclusion :* l'extrait de feuilles de *N. lappaceum* a montré sa capacité à diminuer l'expression protéique de CYR61. L'extrait est donc actif sur la fermeté de la peau et/ou des muqueuses.

### Exemple 6 : Exemple d'ingrédient cosmétique

Les quantités indiquées sont en pourcentage en poids par rapport au poids total de l'ingrédient cosmétique.

### Exemple 6a)

| | |
|---|---|
| Extrait de *N. lappaceum* Ex. 1a) à 1e) | 1-20% (p/p) |
| Maltodextrines | 80-99% (p/p) |
| Exemple 6b) | |
| Extrait de *N. lappaceum* Ex. 1a) à 1e) | 1% |
| Glycérine | 79% |
| Biopropanediol | 10% |
| Eau | 10% |

### Exemple 7 : Exemples de compositions cosmétiques

Les compositions ci-après sont préparées selon des méthodes connues de l'homme du métier, en particulier s'agissant des différentes phases à mélanger entre elles.

*L'ingrédient cosmétique est préparé selon l'exemple 6 précédent. Les quantités indiquées sont en pourcentage en poids par rapport au poids total de la composition.
**7a)**

| | |
|---|---|
| Ingrédient cosmétique* | 0,2-0,3 |
| EDTA | 0,05 |
| Steareth-2 | 2,00 |
| Steareth-21 | 2,50 |
| Alcool cetearyl | 1,00 |
| Caprylate de propylheptyl | 15,00 |
| Hydroxide de sodium (30 % en solution) | 0,10 |
| Mélange de phenoxyéthanol, chlorphenesine, acide benzoique, Butylène glycol, acide sorbique (Germazide ^{™} PBS) | 1,25 |
| Mélange de polyacrylate-X, d'isohexadecane et de polysorbate 60 | |
| (Sepigel^{™} SMS 60) | 4,00 |
| Eau | Qsp 100 |

**7b)**

| | |
|---|---|
| Ingrédient cosmétique* | 0,2-0,3 |
| EDTA | 0,05 |
| Steareth-2 | 2,00 |
| Steareth-21 | 2,50 |
| Alcool cetearyl | 1,00 |
| Caprylate de propylheptyl | 15,00 |
| Hydroxide de sodium (30 % en solution) | 0,10 |
| Mélange de phenoxyéthanol, chlorphenesin, acide benzoique, Butylène glycol, acide sorbique (Germazide ^{™} PBS) | 1,25 |
| Mélange de polyacrylate-X, d'isohexadecane et de polysorbate 60 (Sepigel^{™} SMS 60) | 4,00 |
| Eau | Qsp 100 |

**7c)**

On procède selon les méthodes connues de l'homme du métier pour mélanger ensemble les différentes phases A, B, C, D ci-dessous pour préparer une composition selon la présente invention. Les proportions sont exprimées en %.

| **Phase A** | |
|---|---|
| Glycérile stéarate, PEG-100 Stéarate | 4,00 |
| Pentaerythrityl distéarate | 1,50 |
| Cétéaryl Isononanoate | 3,00 |
| Propylheptyl caprylate | 5,00 |
| Coco caprylate | 2,00 |
| Dicaprylyl carbonate | 3,00 |
| Diméthicone | 1,00 |

| **Phase B** | |
|---|---|
| Eau | 64,43 |
| Propylène glycol, phenoxyéthanol, chlorphenesine, methylparabène | 1,00 |
| Glycérine | 1,57 |
| Gomme de xanthane | 0,20 |
| Butylène glycol | 2,00 |
| Hydroxyde de sodium, eau | 0,15 |

| **Phase C** | |
|---|---|
| Carbomère | 0,15 |
| Eau | 10 |

| **Phase D** | |
|---|---|
| Extrait de *N. lappaceum* obtenu selon exemple 6a) | 1,00 |

## Revendications

1. Utilisation cosmétique d'un extrait de feuilles de *Nephelium lappaceum* pour augmenter la fermeté et/ou l'élasticité de la peau et/ou des muqueuses.

2. Utilisation de l'extrait selon la revendication 1 pour augmenter l'élasticité de la peau et/ou des muqueuses.

3. Utilisation de l'extrait selon la revendication 1 pour augmenter la fermeté de la peau et/ou des muqueuses

4. Utilisation de l'extrait selon l'une quelconque des revendications 1 et 3 à 4 telle que l'extrait augmente l'expression génique et/ou protéique de collagène de type I et/ou V, de préférence de collagène de type I.

5. Utilisation de l'extrait selon l'une quelconque des revendications 1 à 2 et 4, telle que l'extrait augmente l'expression génique et/ou protéique de LOX-L, de fibuline-5, d'Emiline-1 et/ou de fibrilline-1, préférentiellement de fibrilline-1, encore préférentiellement l'expression protéique de fibrilline-1.

6. Utilisation de l'extrait selon l'une quelconque des revendications 1 à 5 telle que l'extrait est obtenu dans un solvant ou un mélange de solvants tel que l'eau, un alcool, un glycol, un polyol, un mélange eau/alcool, eau/glycol ou eau/polyol (tels que l'eau en mélange avec éthanol, glycérol et/ou butylène glycol et/ou autres glycols tel que xylitol et/ou propanediol) de 99/1 à 1/99 (p/p), avantageusement dans l'eau comme unique solvant, plus préférentiellement dans l'eau en conditions subcritiques.

7. Utilisation de l'extrait selon l'une quelconque des revendications 1 à 6 telle que l'extrait est atomisé en présence d'une concentration en poids de maltodextrines comprise entre 20% et 90%, préférentiellement entre 40 et 80%, encore préférentiellement de 70 à 80% par rapport au poids total de la poudre obtenue.

8. Utilisation de l'extrait selon l'une quelconque des revendications 1 à 7, **caractérisée en ce que** l'extrait est appliqué sur tout ou partie du corps, préférentiellement les jambes, les cuisses, les bras, le ventre, le décolleté et/ou le cou ; et/ou tout ou partie du visage, préférentiellement les joues, le front, le menton, les lèvres, le contour des lèvres, le contour des yeux, et/ou la zone dite « en T » du visage.

9. Utilisation de l'extrait selon l'une quelconque des revendications 1 à 8, **caractérisée en ce que** l'extrait est adapté à une application par voie topique.

10. Utilisation selon la revendication 1 pour prévenir et/ou traiter les vergetures.

11. Extrait de feuilles de *Nephelium lappaceum* pour son utilisation par voie topique ou orale, seul ou dans une composition dermatologique ou pharmaceutique le comprenant, dans la prévention et/ou le traitement de pathologies impliquant une perte d'expression protéique et/ou génique de collagène, préférentiellement de collagène de type I et/ou une perte de fermeté pathologique de la peau et/ou des muqueuses choisies parmi couperose et télangiectasies.

12. Extrait de feuilles de *Nephelium lappaceum* pour son utilisation par voie topique ou orale, seul ou dans une composition dermatologique ou pharmaceutique le comprenant, dans la prévention et/ou le traitement de pathologies impliquant une perte d'élasticité pathologique choisies parmi élastose solaire et/ou cutis laxa.

## Patentansprüche

1. Kosmetische Verwendung eines Extrakts aus Blättern von *Nephelium lappaceum* zur Erhöhung der Festigkeit und/oder Elastizität der Haut und/oder der Schleimhäute.

2. Verwendung des Extrakts nach Anspruch 1 zur Erhöhung der Elastizität der Haut und/oder der Schleimhäute.

3. Verwendung des Extrakts nach Anspruch 1 zur Erhöhung der Festigkeit der Haut und/oder der Schleimhäute.

4. Verwendung des Extrakts nach einem der Ansprüche 1 und 3 bis 4 derart, dass der Extrakt die Gen- und/oder Proteinexpression von Kollagen Typ I und/oder V, vorzugsweise von Kollagen Typ I, erhöht.

5. Verwendung des Extrakts nach einem der Ansprüche 1 bis 2 und 4 derart, dass der Extrakt die Gen- und/oder Proteinexpression von LOX-L, Fibulin-5, Emilin-1 und/oder Fibrillin-1, vorzugsweise von Fibrillin-1, noch stärker bevorzugt die Proteinexpression von Fibrillin-1, erhöht.

6. Verwendung des Extrakts nach einem der Ansprüche 1 bis 5 derart, dass der Extrakt in einem Lösungsmittel oder einem Gemisch von Lösungsmitteln, wie Wasser, einem Alkohol, einem Glykol, einem Polyol, einem Wasser/Alkohol-, Wasser/Glykol- oder Wasser/PolyolGemisch (wie Wasser im Gemisch mit Ethanol, Glycerin und/oder Butylenglykol und/oder anderen Glykolen, wie Xylit und/oder Propandiol), von 99/1 bis 1/99 (W/W), vorteilhafterweise in Wasser als einzigem Lösungsmittel, stärker bevorzugt in Wasser, unter subkritischen Bedingungen erhalten wird.

7. Verwendung des Extrakts nach einem der Ansprüche 1 bis 6 derart, dass der Extrakt in Gegenwart einer Konzentration, bezogen auf Gewicht, von Maltodextrinen zwischen 20 Gew.-% und 90 Gew.-%, vorzugsweise zwischen 40 und 80 Gew.-%, noch stärker bevorzugt zwischen 70 und 80 Gew.-%, bezogen auf das Gesamtgewicht des erhaltenen Pulvers, zerstäubt wird.

8. Verwendung des Extrakts nach einem der Ansprüche 1 bis 7, **dadurch gekennzeichnet, dass** der Extrakt auf den gesamten Körper oder einen Teil davon, vorzugsweise auf die Beine, die Oberschenkel, die Arme, den Bauch, das Dekolleté und/oder den Hals, und/oder das gesamte Gesicht oder Teile davon, vorzugsweise die Wangen, die Stirn, das Kinn, die Lippen, die Lippenkontur, die Augenkontur und/oder die so genannte "T"-Zone des Gesichts, aufgebracht wird.

9. Verwendung des Extrakts nach einem der Ansprüche 1 bis 8, **dadurch gekennzeichnet, dass** der Extrakt für die topische Anwendung geeignet ist.

10. Verwendung nach Anspruch 1 zur Vorbeugung und/oder Behandlung von Dehnungsstreifen.

11. Extrakt aus Blättern von *Nephelium lappaceum* zur topischen oder oralen Verwendung, allein oder in einer dermatologischen oder pharmazeutischen Zusammensetzung, die ihn umfasst, bei der Vorbeugung und/oder Behandlung von Pathologien, die einen Verlust der Protein- und/oder -Genexpression von Kollagen, vorzugsweise von Kollagen Typ I, und/oder einen pathologischen Festigkeitsverlust der Haut und/oder der Schleimhäute mit sich bringen, ausgewählt aus Couperose und Teleangiektasie.

12. Extrakt aus Blättern von *Nephelium lappaceum* zur topischen oder oralen Verwendung, allein oder in einer dermatologischen oder pharmazeutischen Zusammensetzung, die ihn umfasst, bei der Vorbeugung und/oder Behandlung von Pathologien, die einen pathologischen Elastizitätsverlust mit sich bringen, ausgewählt aus Sonnenelastase und/oder Cutis laxa.

## Claims

1. The cosmetic use of a *Nephelium lappaceum* leaf extract for increasing the firmness and/or elasticity of the skin and/or mucous membranes.

2. The use of the extract as claimed in claim 1, for increasing the elasticity of the skin and/or mucous membranes.

3. The use of the extract as claimed in claim 1, for increasing the firmness of the skin and/or mucous membranes.

4. The use of the extract as claimed in any one of claims 1 and 3 to 4, such that the extract increases type I and/or type V collagen, preferably type I collagen, gene and/or protein expression.

5. The use of the extract as claimed in any one of claims 1 to 2 and 4, such that the extract increases LOX-L, fibulin-5, emilin-1 and/or fibrillin-1, preferentially fibrillin-1, gene and/or protein expression, more preferentially fibrillin-1 protein expression.

6. The use of the extract as claimed in any one of claims 1 to 5, such that the extract is obtained in a solvent or a mixture of solvents, such as water, an alcohol, a glycol, a polyol, a water/alcohol, water/glycol or water/polyol mixture (such as water mixed with ethanol, glycerol and/or butylene glycol and/or other glycols such as xylitol and/or propanediol), from 99/1 to 1/99 (w/w), advantageously in water as sole solvent, more preferentially in water under subcritical conditions.

7. The use of the extract as claimed in any one of claims 1 to 6, such that the extract is spray-dried in the presence of a concentration by weight of maltodextrins of between 20% and 90%, preferentially between 40% and 80%, more preferentially from 70% to 80% relative to the total weight of the powder obtained.

8. The use of the extract as claimed in any one of claims 1 to 7, wherein the extract is applied to all or part of the body, preferentially the legs, thighs, arms, stomach, bust and/or neck; and/or all or part of the face, preferentially the cheeks, forehead, chin, lips, area around the lips, area around the eyes, and/or the "T" zone of the face.

9. The use of the extract as claimed in any one of claims 1 to 8, wherein the extract is suitable for topical application.

10. The use as claimed in claim 1, for preventing and/or treating stretch marks.

11. *A Nephelium lappaceum* leaf extract for topical or oral use thereof, alone or in a dermatological or pharmaceutical composition comprising it, in the prevention and/or treatment of pathological conditions involving a loss of collagen protein and/or gene expression, preferentially type I collagen protein and/or gene expression, and/or a pathological loss of firmness of the skin and/or mucous membranes, chosen from rosacea and telangiectasia.

12. A *Nephelium lappaceum* leaf extract for topical or oral use thereof, alone or in a dermatological or pharmaceutical composition comprising it, in the prevention and/or treatment of pathological conditions involving a pathological loss of elasticity, chosen from solar elastosis and/or cutis laxa.
